# FASCICULE DE BREVET EUROPEEN

(11) **EP 3 892 906 B1**
(45) Date de publication et mention de la délivrance du brevet: **09.10.2024**
(21) Numéro de dépôt: 21151916.0
(22) Date de dépôt: 15.01.2021
(51) Int. Cl.: F16L 29/02, A61M 16/08, A61M 39/26, F16K 15/02, F16L 37/407, F16L 37/60, A61M 1/00, A61M 16/20, A61M 39/24

(54) **PRISE DE DISTRIBUTION DE FLUIDE À CHAMBRE À BILLE-CLAPET EXTRACTIBLE UTILISABLE EN MILIEU HOSPITALIER**
FLUIDVERTEILUNGSANSCHLUSS MIT HERAUSZIEBARER VENTILKUGELKAMMER ZUR VERWENDUNG IN EINER KRANKENHAUSUMGEBUNG
FLUID DISTRIBUTION SOCKET WITH CHAMBER WITH REMOVABLE BALL-VALVE FOR USE IN HOSPITALS

(30) Priorité: 10.04.2020 FR 2003655
(43) Date de publication de la demande: 13.10.2021
(73) Titulaire: Air Liquide Medical Systems, 92160 Antony (FR)
(72) Inventeur: CHEVEREAU, Christophe, 92400 Courbevoie (FR); BEAUCHER, Jérôme, 92160 Antony (FR); RUDNIANYN, Philippe, 92160 Antony (FR); FAVRE REGUILLON, Loic, 92160 Antony (FR)
(74) Mandataire: Air Liquide

(56) Documents cités:
- EP-A1- 1 611 921
- FR-A1- 2 628 820
- FR-A1- 2 995 654
- US-A1- 2003 084 663

## Description

L'invention concerne une prise de distribution de fluide, en particulier de gaz médical ou de vide (i.e. dépression) comprenant une chambre ou cage à bille-clapet extractible, c'est-à-dire pouvant être facilement montée et démontée, et un guide-embout aussi extractible, ladite prise de distribution de fluide étant configurée pour être utilisée dans un bâtiment hospitalier ou analogue, typiquement une prise murale fixée directement ou indirectement à une paroi verticale, tel un mur ou analogue, pour fournir du gaz ou du vide, notamment dans les chambres, les salles d'opération ou de soins, ou autres au sein du bâtiment hospitalier.

Les prises de distribution de fluide sont utilisées pour distribuer les fluides, en particulier les gaz médicaux (i.e. un gaz pur ou un mélange gazeux) ou le vide (i.e. dépression < 1 atm), au sein des bâtiments hospitaliers ou analogues. Les prises de distribution de fluide sont couramment appelées « prises murales » ou « raccords muraux » car elles sont généralement montées soit directement sur une paroi, c'est-à-dire un mur ou analogue, d'un bâtiment hospitalier, soit indirectement, par exemple en étant intégrées à un boitier ou analogue qui est lui-même monté sur une paroi, en particulier dans les chambres des patients, dans les salles d'opération ou de soins, notamment les salles de réanimation, ou d'autres pièces

Les prises murales permettent de fournir les gaz médicaux véhiculés par les réseaux de canalisations de gaz parcourant les bâtiments hospitaliers, aux appareils et équipements utilisés pour traiter et soigner les patients au sein de ces bâtiments, en particulier les gaz thérapeutiques, tel que l'oxygène, le protoxyde d'azote ou l'air, ou le vide médical (i.e. dépression) permettant d'opérer des aspirations notamment de liquides biologiques, par exemple le sang ou d'autres liquides biologiques.

Ainsi, le document FR-A-2628820 propose une prise de distribution de fluide, appelée raccord à verrouillage automatique, comprenant un corps de prise de forme allongée comprenant une cavité ou passage central traversant axialement le corps de prise de sorte de relier fluidiquement une extrémité amont, aussi appelée extrémité d'entrée ou extrémité distale, à une extrémité aval, aussi appelée extrémité de sortie ou extrémité proximale comprenant un orifice de fourniture de fluide, c'est-à-dire de gaz ou de vide. Le fluide « circule » dans la prise, lorsqu'un connecteur d'un appareil ou équipement médical, notamment le connecteur d'une conduite de gaz, est raccordé mécaniquement et fluidiquement à la prise.

Des éléments de contrôle du passage de fluide internes à la prise murale permettent de contrôler la libération du gaz, en particulier d'empêcher toute délivrance du gaz quand aucun connecteur s'y est branché.

Ainsi, une prise murale comprend en général un guide-embout extractible agencé du côté proximal de la prise, c'est-à-dire vers l'avant de la prise comprenant l'orifice de sortie de gaz ou d'aspiration pour le vide, et une chambre à bille-clapet du côté distal de la prise, c'est-à-dire vers l'arrière ou le fond de la prise. Le guide-embout comprend un clapet de tête coulissant coopérant avec un siège de clapet pour assurer ou, à l'inverse, interrompre l'étanchéité fluidique entre eux et ainsi empêcher ou, à l'inverse, autoriser le passage de fluide, i.e. gaz ou vide, lorsqu'un connecteur d'un appareil ou équipement médical est raccordé à la prise murale. Par ailleurs, la chambre à bille-clapet sert à empêcher toute circulation de fluide, i.e. gaz ou vide, lorsque le guide-embout est extrait du corps de prise, ou à diminuer le débit de fuite.

Ce type de prise murale doit subir régulièrement des opérations de maintenance, notamment afin de garantir leur bon fonctionnement, de remplacer les éléments usés ou détériorés (e.g. utilisation intensive), de les nettoyer.... Lors de ces opérations de maintenance, il est nécessaire de pouvoir démonter puis remonter facilement et rapidement les différents éléments internes de la prise murale, en particulier le guide-embout situé vers l'avant du corps de prise et la chambre à bille-clapet.

Or, la chambre à bille-clapet, aussi appelée ou cage à bille-clapet, qui est située derrière le guide-embout, c'est-à-dire au fond du corps de prise, donc dans un endroit exigu, est difficile d'accès et offre peu de prise aux outils de démontage, ce qui rend la maintenance difficile.

Le problème est donc de pouvoir démonter et remonter facilement la chambre à bille-clapet d'une prise de distribution de fluide, i.e. de gaz ou de vide, telle une prise murale utilisée en milieu hospitalier, en particulier lors d'une opération de maintenance de la prise.

La solution concerne alors une prise de distribution de fluide, typiquement une prise murale, pour distribuer un fluide au sein d'un bâtiment hospitalier, comprenant un corps de prise de forme allongée, d'axe longitudinal AA, traversé axialement par un passage central formant un logement interne au sein duquel est agencé un guide-embout extractible logeant un clapet de tête mobile, et une chambre à bille-clapet extractible, le guide-embout extractible comprenant en outre une tige axiale faisant saillie sur la surface externe arrière du guide-embout et étant solidaire dudit guide-embout, et ladite chambre à bille-clapet extractible comprenant :
- une paroi périphérique délimitant un compartiment interne contenant une bille-clapet mobile, et portant un filetage périphérique externe aménagé autour de ladite paroi périphérique la bille-clapet mobile ayant une forme sphérique ou comprenant une base allongée surmontée d'une tête hémisphérique,
- une première extrémité avec un passage de fluide en communication fluidique avec le compartiment interne par un orifice intérieur et avec l'extérieur de la chambre à bille-clapet par un orifice extérieur, et
- une seconde extrémité avec une ouverture large en communication fluidique avec le compartiment interne,
   et dans laquelle le guide-embout vient appuyer sur la bille-clapet de la chambre à bille-clapet extractible via la tige axiale faisant saillie sur la surface externe arrière du guide-embout, au travers du passage de fluide axial,
   dans laquelle :
      - le passage central du corps de prise comprend un premier taraudage coopérant avec le filetage périphérique aménagé dans la paroi périphérique de la chambre à bille-clapet pour permettre un montage par vissage de la chambre à bille-clapet dans le corps de prise, et
      - le passage de fluide axial agencé dans la première extrémité de la chambre à bille-clapet extractible est conformé pour présenter une section non-circulaire sur un tronçon d'entrée s'étendant depuis l'orifice extérieur du passage de fluide axial et en direction de l'orifice intérieur du passage de fluide axial, de manière à permettre le vissage de la chambre à bille-clapet au sein de la prise de distribution de fluide, au moyen d'un outil présentant une forme complémentaire de la section non circulaire, et
      - la paroi périphérique de la chambre à bille-clapet extractible est conformée pour présenter des expansions de parois agencées au niveau de la seconde extrémité, lesdites expansions de parois faisant saillie en éloignement par rapport à ladite paroi périphérique et étant repliées radialement vers l'axe longitudinal (AA) de manière à y retenir la bille-clapet mobile.

Une telle structure permet un montage facile et rapide de la chambre à bille-clapet amovible par vissage ou dévissage au sein d'une prise de distribution de fluide, au moyen d'un outil présentant une forme complémentaire de la section non circulaire, en particulier polygonale, de préférence hexagonale, du passage de fluide axial. Grâce à cela, les opérations de maintenance opérées régulièrement sur la prise murale sont plus rapides et plus aisées.

Selon le mode de réalisation considéré, la prise de distribution de fluide de l'invention peut comprendre l'une ou plusieurs des caractéristiques suivantes :
- la prise de distribution de fluide est configurée pour être montée/solidarisée, directement ou indirectement à une paroi d'un bâtiment hospitalier ou analogue, en particulier des moyens de fixation murale.
- la prise de distribution de fluide comprend des moyens de fixation murale permettant sa fixation à une paroi.
- la prise de distribution de fluide comprend des moyens de fixation murale permettant sa fixation et/ou son intégration à un caisson de distribution de fluide, en particulier un caisson de distribution venant lui-même se fixer à une paroi du bâtiment hospitalier.
- la surface externe arrière du guide-embout est agencée face à la chambre à bille-clapet extractible.
- la tige axiale du guide-embout fait saillie en direction de la chambre à bille-clapet.
- la tige axiale du guide-embout est agencée axialement selon l'axe longitudinal (AA).
- la chambre à bille-clapet et le guide-embout sont extractibles, i.e. amovibles, c'est-à-dire qu'ils peuvent être montés et démontés du passage central formant le logement interne du corps de prise.
- la prise de distribution de fluide comprend un orifice proximal, aussi appelé orifice ou ouverture avant, en communication fluidique avec le passage central formant le logement interne du corps de prise.
- la chambre à bille-clapet et le guide-embout sont conformés pour être extractibles du corps de prise par ledit orifice proximal.
- le passage de fluide de la chambre à bille-clapet extractible est conformé pour présenter une section polygonale sur le tronçon d'entrée.
- le passage de fluide de la chambre à bille-clapet est conformé pour présenter une section hexagonale sur le tronçon d'entrée, en particulier une structure de type 6-pans.
- le tronçon d'entrée du passage de fluide de la chambre à bille-clapet a une longueur de 0,5 à 5 mm, typiquement de 1 à 3 mm, par exemple de l'ordre de 2 mm.
- le passage de fluide de la chambre à bille-clapet est aménagé coaxialement à un axe longitudinal (AA) de la chambre à bille-clapet.
- le passage de fluide, l'orifice intérieur et l'orifice extérieur sont coaxiaux et agencés sur l'axe longitudinal (AA).
- la paroi périphérique de la chambre à bille-clapet comprend de 2 à 6 expansions de parois agencées à la seconde extrémité, typiquement de 2 à 4 expansions de parois, par exemple 2 ou 3 expansions.
- les expansions de parois de la chambre à bille-clapet forment des languettes ou analogues.
- l'ouverture large de la chambre à bille-clapet comprend une bordure circulaire, les expansions de parois étant portées par ladite bordure circulaire et formées d'une pièce avec ladite bordure circulaire.
- la bille-clapet (pour prise de fourniture de gaz) est une bille sphérique.
- alternativement, la bille-clapet (pour prise de fourniture de vide, i.e. de dépression) comprend une base allongée surmontée d'une demi-sphère, c'est-à-dire qu'elle a une forme générale de champignon.
- la bille-clapet est en métal, par exemple en acier.
- le compartiment interne contenant la bille-clapet mobile comprend une paroi interne conformée pour présenter un siège de bille-clapet coopérant avec la bille-clapet pour contrôler le passage de fluide.
- la chambre à bille-clapet comprend un corps creux, i.e. un corps tubulaire, comprenant la paroi périphérique délimitant le compartiment interne.
- la chambre à bille-clapet a une forme générale tubulaire.
- la chambre à bille-clapet, en particulier son corps creux, a une forme générale tubulaire et comprend une paroi avant formant la première extrémité, c'est-à-dire une forme générale de coupelle.
- la chambre à bille-clapet a une forme générale de coupelle comprenant un corps creux de forme générale tubulaire et une paroi avant formant la première extrémité traversée par le passage de fluide.
- la paroi avant ferme la première extrémité du corps creux de la chambre à bille-clapet à la manière d'un couvercle ou d'un fond.
- la paroi avant et le corps creux et/ou les expansions de paroi de la chambre à bille-clapet sont formés d'une seule pièce.
- le corps tubulaire et/ou la paroi avant et/ou les expansions de paroi de la chambre à bille-clapet sont en métal ou alliage métallique, de préférence en laiton.
- le tronçon d'entrée de la chambre à bille-clapet s'étend sur plus de la moitié de la longueur du passage de fluide axial, de préférence sur toute la longueur du passage de fluide axial.
- la chambre à bille-clapet, en particulier son corps creux, a une longueur totale de 16 à 20 mm, typiquement de l'ordre de 18 mm.
- le tronçon d'entrée du passage de fluide axial de la chambre à bille-clapet a une aire de section de l'ordre de 20 à 35 mm², de préférence de l'ordre de 21 à 25 mm².
- la chambre à bille-clapet et ses éléments forment un premier ensemble extractible.
- le guide-embout et ses éléments forment un second ensemble extractible.
- le guide-embout extractible loge un clapet de tête mobile.
- le guide-embout est agencé/situé dans le passage central formant le logement interne du corps de prise entre la chambre à bille-clapet et l'orifice proximal.
- le guide-embout comprend un logement axial à fond borgne.
- le guide-embout comprend par ailleurs un élément élastique, tel un ressort cylindrique ou analogue, agencé dans le logement axial du guide-embout, de préférence dans le fond borgne du logement axial.
- l'élément élastique du guide-embout est agencé entre le clapet de tête et le fond borgne du logement axial du guide-embout.
- l'élément élastique du guide-embout vient prendre appui sur le fond borgne du logement axial.
- l'élément élastique du guide-embout vient prendre appui, d'autre part, sur le clapet de tête, en particulier sur un épaulement annulaire solidaire du clapet de tête, par exemple formé dans la paroi périphérique externe du clapet de tête.
- l'élément élastique du guide-embout est agencé autour d'une portion arrière du clapet de tête.
- l'élément élastique du guide-embout est agencé pour normalement repousser le clapet de tête contre un siège de clapet aménagé dans le guide-embout, de préférence au sein de la paroi interne délimitant le logement axial du guide-embout, de sorte de contrôler le passage de fluide dans le corps de prise.
- le guide-embout comprend aussi au moins un élément d'étanchéité permettant d'assurer une étanchéité fluidique, lorsque le clapet de tête est repoussé contre le siège de clapet par l'élément élastique.
- le passage central du corps de prise comprend un premier taraudage coopérant avec le filetage périphérique (i.e. un premier filetage) aménagé dans la paroi périphérique de la chambre à bille-clapet pour permettre un montage par vissage, i.e. une solidarisation par vissage, de la chambre à bille-clapet dans le corps de prise.
- le guide-embout extractible logeant le clapet de tête mobile comprend par ailleurs un second filetage coopérant avec un second taraudage aménagé dans le passage central du corps de prise pour permettre un montage par vissage, i.e. une solidarisation par vissage, du guide-embout dans le corps de prise.
- le guide-embout et/ou la chambre à bille-clapet peuvent être extraits du passage central du corps de prise par dévissage.
- la prise de distribution de fluide comprend en outre des moyens de raccordement fluidique à une canalisation de fluide, i.e. de gaz ou de vide.
- les moyens de raccordement fluidique comprennent une tubulure.
- la tubulure comprend un lumen en communication fluidique avec, d'une part, la canalisation de fluide et, d'autre part, le passage central interne de la prise de distribution de fluide.

L'invention concerne aussi une utilisation d'une prise de distribution de fluide selon l'invention pour distribuer un fluide au sein d'un bâtiment hospitalier, ladite prise de distribution de fluide étant montée directement (i.e. à même de la paroi) ou indirectement (i.e. via un coffret de distribution de fluide elle-même montée sur la paroi), sur une paroi du bâtiment hospitalier et raccordée fluidiquement à une canalisation de gaz ou de vide agencée au sein dudit bâtiment hospitalier.

Selon un autre aspect, l'invention porte aussi sur un tel bâtiment hospitalier comprenant :
- au moins une canalisation de gaz ou de vide agencée au sein dudit bâtiment hospitalier pour y acheminer du gaz ou du vide,
- au moins une pièce comprenant une paroi, tel un mur,
- et au moins une prise de distribution de fluide selon l'invention montée sur ladite paroi et raccordée fluidiquement à ladite au moins une canalisation de gaz ou de vide.

Ladite au moins une canalisation de gaz ou de vide fait partie d'un réseau de canalisations de fluide parcourant le bâtiment hospitalier et alimentant une ou plusieurs pièces de ce bâtiment hospitalier, en particulier une ou des chambres, salles de soins, d'opérations, de réveil ou autres.

La prise est montée directement à une paroi ou indirectement via un coffret de distribution de fluide lui-même fixé à une paroi, typiquement un mur.

Ladite au moins une canalisation de fluide comprend au moins une canalisation d'amenée d'un gaz choisi parmi l'oxygène, l'air médical ou le protoxyde d'azote (y compris les mélanges N₂O/O₂). Ladite au moins une canalisation d'amenée de gaz est alimentée par un ou des réservoirs de fluide contenant ledit (lesdits) gaz sous forme liquide et/ou gazeuse.

Alternativement, ladite au moins une canalisation de fluide comprend au moins une canalisation d'amenée de vide (i.e. de dépression, e.g. pression < 1 atm) reliée fluidiquement à des moyens de mise sous vide, telle une ou des pompes à vide.

L'invention va maintenant être mieux comprise grâce à la description détaillée suivante, faite à titre illustratif mais non limitatif, en référence aux figures annexées parmi lesquelles :
Fig. 1 représente un mode de réalisation d'une prise murale de distribution de gaz médical équipée d'une chambre à bille-clapet selon l'invention,
Fig. 2 représente un mode de réalisation d'une prise murale de distribution de vide (i.e.dépression) équipée d'une chambre à bille-clapet selon l'invention,
Fig. 3 est une vue en coupe longitudinale d'une chambre à bille-clapet pour une prise murale de distribution de gaz médical selon l'invention,
Fig. 4 est une vue de face de la chambre à bille-clapet de Fig. 3,
Fig. 5 est une vue en coupe longitudinale d'une chambre à bille-clapet pour une prise murale de distribution de vide selon l'invention, et
Fig. 6 est une vue de face de la chambre à bille-clapet de Fig. 5.

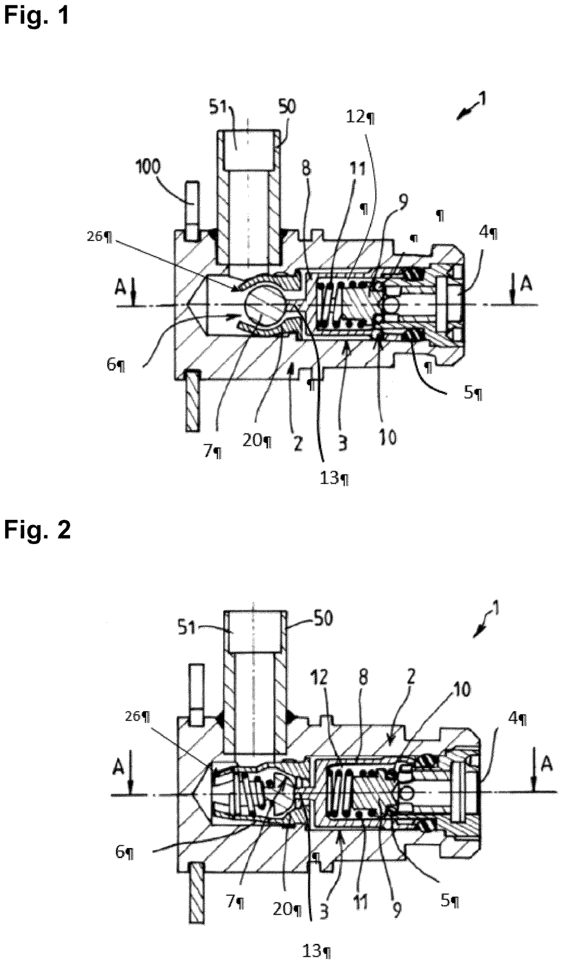

Fig. 1 représente un mode de réalisation d'une prise de distribution 1 de gaz selon l'invention, par exemple d'oxygène ou d'air médical, comprenant un corps de prise 2 de forme allongée, selon un axe longitudinal AA, lequel est traversé axialement par un passage central 3 formant un logement interne au sein duquel sont agencés des éléments de contrôle du passage de gaz.

Les éléments de contrôle du passage de gaz comprennent un guide-embout 8 extractible comprenant un logement axial 12, un clapet de tête 9 mobile selon l'axe AA, i.e. coulissant en translation, au sein dudit logement axial 12 et un orifice proximal 4. Dans le cas de la prise 1 de Fig. 1, le gaz est distribué, c'est-à-dire sort de la prise 1, par l'orifice proximal 4 lorsqu'on appareil ou équipement y est raccordé, tel un conduit flexible, via un connecteur adapté. L'orifice proximal 4 est de section circulaire.

Le guide-embout 8 comprend par ailleurs un élément élastique 11, tel un ressort cylindrique ou analogue, agencé dans le logement axial 12 du guide-embout 8, de préférence dans le fond borne du logement axial 12.

De préférence, l'élément élastique 11 est agencé autour de la portion arrière du clapet de tête 9 et vient prendre appui, d'une part, sur le fond borgne du logement axial 12 et, d'autre part, sur un épaulement annulaire solidaire du clapet de tête 9, par exemple formé dans la paroi périphérique externe du clapet de tête 9. L'élément élastique 11 permet de normalement repousser le clapet de tête 9 contre un siège de clapet 10 aménagé dans le guide-embout 8, par exemple au sein de la paroi interne 8a délimitant le logement axial 12 du guide-embout 8, de sorte de contrôler le passage de fluide dans le corps de prise 2.

Par ailleurs, le guide-embout 8 comprend aussi au moins un élément d'étanchéité 5 permettant d'assurer une étanchéité fluidique, lorsque le clapet de tête 9 est repoussé contre le siège de clapet 10 par l'élément élastique 11.

Par ailleurs, la prise 1 de distribution de fluide comprend aussi un système de sécurité agencé au fond du passage central 3, c'est-à-dire au fond du corps de prise 2, permettant d'empêcher ou limiter la circulation de gaz en direction de l'orifice de sortie 4, lorsque le guide-embout 8 est extrait du corps de prise 2, par exemple lors d'une opération de maintenance du guide-embout 8.

Ce système de sécurité comprend une chambre à bille-clapet 6 dans laquelle est logée une bille-clapet 7, à savoir ici une bille de forme sphérique, coopérant avec un siège de bille-clapet situé dans la chambre à bille-clapet 6. La chambre à bille-clapet 6 est extractible du corps de prise 2 afin de permettre sa maintenance.

Ce système de sécurité permet d'empêcher que le gaz ne puisse s'échapper de la prise 1 ou que l'aspiration par le vide (i.e. dépression) ne se fasse, c'est-à-dire que de l'air ne puisse entrer dans le réseau de vide relié à la prise 1 et y faire remonter la pression (< 1 atm) qui y règne, lorsque les éléments de contrôle du passage de gaz, typiquement le guide-embout 8, sont démontés et extraits du corps de prise 2, notamment lors d'une opération de vérification, de maintenance ou de remplacement.

La bille-clapet 7 fait office de clapet de sécurité venant, sous l'effet de la pression fluidique ou de la dépression (i.e. vide) s'exerçant sur la bille-clapet 7 en cas de démontage du guide-embout 8, appuyer sur et obturer un siège de clapet situé dans la chambre 6. A l'inverse, quand les éléments de contrôle du passage de gaz sont montés dans le corps de prise 2, le guide-embout 8 vient appuyer sur la bille-clapet 7 pour la décoller du siège de clapet 22 et autoriser ainsi la communication fluidique au travers du canal de liaison (i.e. le passage de fluide 22 ; voir ci-dessous). Ceci peut se faire via une tige axiale 13 faisant saillie sur la surface externe arrière du guide-embout 8 et solidaire de celui-ci, qui est orientée de sorte de traverser axialement le canal de liaison (i.e. le passage de fluide 22 ; voir ci-dessous) pour venir appuyer sur la bille-clapet 7 et ainsi la décoller de son siège.

Fig. 2 représente un mode de réalisation d'une prise 1 de distribution de vide, c'est-à-dire de dépression selon l'invention, dont l'architecture globale est très similaire à celle de la prise 1 de distribution de gaz de la Fig. 1. Dès lors, les mêmes références ont été utilisées pour désigner des éléments identiques ou similaires et/ou remplissant la même fonction ; ceux-ci ne seront donc pas détaillés ci-après.

Une différence majeure réside toutefois dans le fait que, dans la prise 1 de distribution de vide de la Fig. 2, l'aspiration du vide (i.e. dépression), c'est-à-dire la circulation du gaz aspiré au travers de la prise 1, se fait dans le sens opposé à celui de la circulation de gaz au sein de la prise 1 de Fig. 1. Ainsi, le gaz (e.g. air) est aspiré par la dépression régnant dans le corps de prise 2 par l'orifice proximal 4, ce qui permet d'obtenir un effet de succion, c'est-à-dire d'aspiration, en amont de la prise 1, c'est-à-dire dans un dispositif médical (non montré) raccordé fluidiquement à la prise 1. L'orifice proximal 4 sert donc à aspirer du gaz et non pas à en distribuer.

Une conséquence de cette circulation inverse du fluide est que, dans la prise 1 de distribution de vide de la Fig. 2, la chambre à bille 6 est orientée à l'inverse de celle de la Fig. 1 et que par ailleurs, la bille-clapet 7 n'est pas complètement sphérique mais comprend uniquement ici une tête hémisphérique sur laquelle vient appuyer la tige 13 du guide-embout 8 au travers du canal de liaison (i.e. le passage de fluide 22 ; voir ci-dessous), comme expliqué ci-avant.

Les prises de distribution 1 de fluide des FIG. 1 et FIG. 2 peuvent être montées et fixées via des moyens de fixation murale soit directement sur une paroi (non montrée), c'est-à-dire un mur ou analogue, d'un bâtiment hospitalier, tel un étrier de fixation 100, soit être intégrées à un boitier ou analogue qui est lui-même monté sur la paroi.

Ces prises 1 permettent de fournir le vide ou les gaz médicaux véhiculés par le réseau de canalisations de fluide parcourant le bâtiment hospitalier, aux appareils et équipements utilisés pour traiter et soigner les patients au sein de ces bâtiments. Pour ce faire, elles peuvent être raccordées fluidiquement au réseau de canalisations de fluide d'un tel bâtiment, via une tubulure arrière 50, tel un raccord ou analogue, en communication fluidique, via son lumen 51, avec, d'une part, ledit réseau de canalisations de fluide et, d'autre part, avec le passage central interne 3 de la prise 1.

Selon la présente invention, afin de pouvoir la démonter et la remonter facilement la chambre 6 à bille-clapet d'une prise de distribution de fluide 1, i.e. de gaz ou de vide, en particulier une prise murale utilisée en milieu hospitalier, qu'elle soit une prise 1 de gaz selon la Fig. 1 ou de vide selon la Fig. 2, il est proposé une chambre à bille-clapet 6 extractible, d'axe longitudinal AA, comprenant un corps creux de forme générale tubulaire comprenant une paroi périphérique 20 délimitant un compartiment interne 21 contenant une bille-clapet mobile 7 et un siège de bille-clapet mobile 7 permettant de contrôler le passage de fluide.

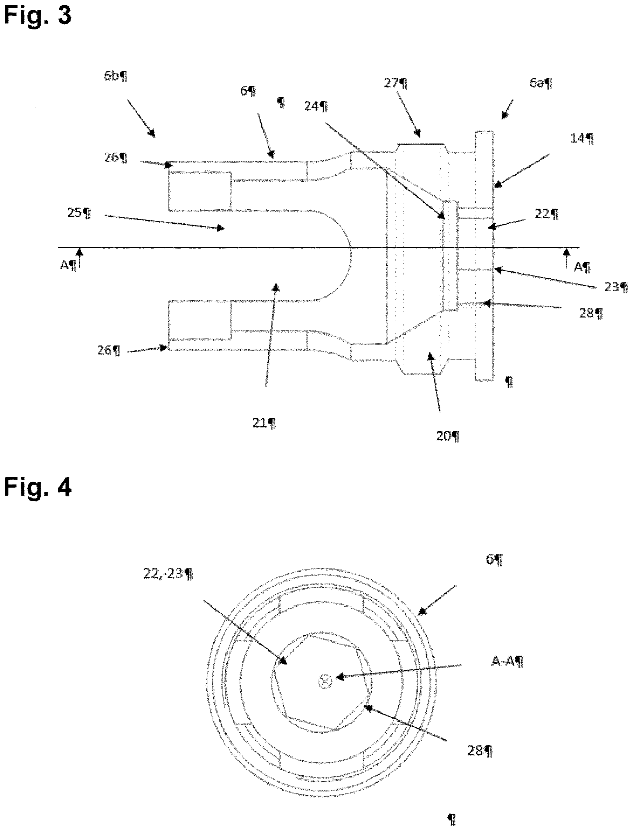

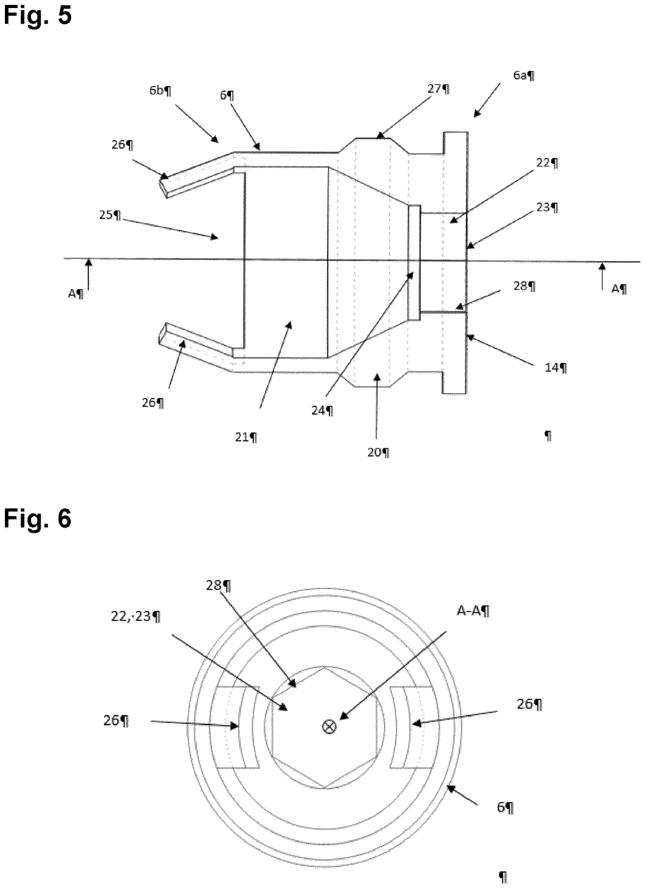

La chambre à bille-clapet 6 comprend en outre une première extrémité 6a et une deuxième extrémité 6b, situées de part et d'autre du compartiment interne 21, c'est-à-dire aux extrémités du corps creux de forme générale tubulaire, comme illustré sur les Fig. 3 et Fig. 5 ; sur ces Figures, la bille-clapet 7 n'est pas représentée.

La première extrémité 6a de la chambre à bille-clapet 6 comprend un passage de fluide 22 axial (i.e. le canal de liaison susmentionné) en communication fluidique avec le compartiment interne 21 par un orifice intérieur 24 et avec l'extérieur par un orifice extérieur 23. Autrement dit, le passage de fluide 22 axial s'étend entre l'orifice extérieur 23 et l'orifice intérieur 24, ce dernier débouchant dans le compartiment interne 21. C'est au travers de ce passage de fluide 22 axial que passe la tige 13 du guide-embout 8, lorsque la chambre à bille-clapet 6 et le guide-embout 8 sont montés dans le corps de prise 2.

La seconde extrémité 6b de la chambre à bille-clapet 6 comprend une ouverture large 25 en communication fluidique avec le compartiment interne 4, c'est-à-dire mettant le compartiment interne 4 en communication fluidique avec l'extérieur.

Autrement dit, la chambre à bille-clapet 6 a une forme générale de coupelle comprenant une paroi périphérique 20 tubulaire avec une paroi avant 14 fermant sa première extrémité 6a, laquelle paroi avant 14 est traversée par le passage de fluide 22 axial, et comprend par ailleurs une ouverture large 25 à sa seconde extrémité 6b.

La paroi périphérique 20 de la chambre à bille-clapet 6 est conformée pour présenter des expansions de parois 26, telles des languettes ou analogues, agencées à la seconde extrémité 6b de ladite paroi périphérique 20, i.e. du corps tubulaire creux, au niveau de l'ouverture 25. Lesdites expansions de parois ou languettes 26 faisant saillie en éloignement par rapport à la paroi périphérique 20 de la chambre à bille-clapet 6 et sont repliées radialement, c'est-à-dire sensiblement vers l'axe AA, de manière à y retenir la bille-clapet 7 lorsqu'elle s'y trouve.

Sur les Fig. 3 et Fig. 4, les expansions de parois ou languettes 26 sont montrées avant pliage radial, c'est-à-dire avant insertion de la bille-clapet 7 au sein du compartiment interne 21, alors que sur les Fig. 1, Fig. 2, Fig. 5 et Fig. 6 les expansions de parois ou languettes 26 sont montrées radialement repliées au niveau de l'ouverture large 25. La section de l'ouverture large 25 est suffisamment large pour permettre l'insertion de la bille-clapet 7 dans le compartiment interne 21, via l'ouverture 25, avant repli des languettes 26, donc supérieure au diamètre de la bille-clapet 7, par exemple elle est de l'ordre de 9 à 12 mm.

Afin de permettre le montage/démontage de la chambre 6 à bille-clapet 7 par vissage/dévissage au sein de la prise de distribution de gaz 1, un filetage 27 est aménagé extérieurement autour de la paroi périphérique 20, c'est-à-dire sur une partie de sa périphérie externe tubulaire.

Par ailleurs, le passage de fluide axial 22 agencé dans la première extrémité 6a de la chambre 6 à bille-clapet est conformé pour présenter une section non-circulaire, en particulier polygonale, sur son tronçon d'entrée 28 s'étendant depuis l'orifice extérieur 23 du passage de fluide axial 22 et en direction de l'orifice intérieur 24 du passage de fluide axial 22, de préférence une section hexagonale de type à 6 pans, comme illustré sur les Fig. 4 et Fig. 6.

Une telle structure permet un montage facile et rapide de la chambre à bille-clapet 1 par vissage ou dévissage au sein d'une prise de distribution de fluide 1, telles celles des Fig. 1 et Fig. 2, au moyen d'un outil présentant une forme complémentaire du tronçon 28 de section non circulaire, en particulier polygonale, de préférence hexagonale, par exemple une clé de montage/démontage de type clé à 6-pans.

En effet, utiliser une clé à 6-pans venant se loger dans le tronçon 28 de forme complémentaire, c'est-à-dire de section hexagonale, permet, contrairement à un tournevis venant se loger dans une simple fente, de limite les turbulences du gaz du fait de cette forme qui est proche d'une section circulaire et par ailleurs un démontage/remontage aisé de la chambre à bille-clapet 1 par vissage ou dévissage.

Afin de permettre le montage/démontage de la chambre 6 à bille-clapet par vissage/dévissage au sein de la prise de distribution de fluide 1, ladite prise de distribution de fluide 1 comprend un taraudage interne complémentaire du filetage 27 aménagé autour de la paroi périphérique 20 de la chambre 6 à bille-clapet.

La chambre 6 à bille-clapet est formée d'un métal ou alliage métallique, tel du laiton, ou d'un matériau thermoplastique et/ou thermodurcissable.

D'une façon générale, les prises 1 de l'invention sont conçues pour permettre de fournir soit des gaz thérapeutiques, i.e. gaz médicaux, en particulier de l'oxygène, du protoxyde d'azote, de l'air ou tout autre gaz ou mélange gazeux (cf. prise de Fig. 1), soit du vide médical (i.e. une dépression) servant à opérer des aspirations notamment de liquides biologiques, par exemple le sang ou d'autres liquides biologiques (cf. prise de Fig. 2).

Les prises 1 de l'invention sont généralement agencées sur des parois, tels des murs ou analogues, au sein des bâtiments hospitaliers.

## Revendications

1. Prise de distribution de fluide (1) pour distribuer un fluide au sein d'un bâtiment hospitalier comprenant un corps de prise (2) de forme allongée, d'axe longitudinal AA, traversé axialement par un passage central (3) formant un logement interne au sein duquel est agencé un guide-embout (8) extractible logeant un clapet de tête (9) mobile, et une chambre à bille-clapet (6) extractible, le guide-embout (8) extractible comprenant en outre une tige axiale (13) faisant saillie sur la surface externe arrière du guide-embout (8) et étant solidaire dudit guide-embout (8), et ladite chambre à bille-clapet (6) extractible comprenant :
- une paroi périphérique (20) délimitant un compartiment interne (21) contenant une bille-clapet mobile (7), et portant un filetage périphérique externe (27) aménagé autour de ladite paroi périphérique (20), la bille-clapet mobile (7) ayant une forme sphérique ou comprenant une base allongée surmontée d'une tête hémisphérique,
- une première extrémité (6a) avec un passage de fluide (22) en communication fluidique avec le compartiment interne (21) par un orifice intérieur (24) et avec l'extérieur de la chambre à bille-clapet (6) par un orifice extérieur (23), et
- une seconde extrémité (6b) avec une ouverture large (25) en communication fluidique avec le compartiment interne (4),
et dans laquelle :
- le guide-embout (8) vient appuyer sur la bille-clapet (7) de la chambre à bille-clapet (6) extractible via la tige axiale (13) faisant saillie sur la surface externe arrière du guide-embout (8), au travers du passage de fluide axial (22), et
- la paroi périphérique (20) de la chambre à bille-clapet (6) extractible est conformée pour présenter des expansions de parois (26) agencées au niveau de la seconde extrémité (6b), lesdites expansions de parois (26) faisant saillie en éloignement par rapport à ladite paroi périphérique (20) et étant repliées radialement vers l'axe longitudinal (AA) de manière à y retenir la bille-clapet mobile (7), et
- le passage central du corps de prise (2) comprend un premier taraudage coopérant avec le filetage périphérique (27) aménagé dans la paroi périphérique (20) de la chambre à bille-clapet (6) pour permettre un montage par vissage de la chambre à bille-clapet (6) dans le corps de prise (2),
**caractérisée en ce que**
- le passage de fluide axial (22) agencé dans la première extrémité (6a) de la chambre à bille-clapet (6) extractible est conformé pour présenter une section non-circulaire sur un tronçon d'entrée (28) s'étendant depuis l'orifice extérieur (23) du passage de fluide axial (22) et en direction de l'orifice intérieur du passage (24) de fluide axial (22), de manière à permettre le vissage de la chambre à bille-clapet (6) au sein de la prise de distribution de fluide, au moyen d'un outil présentant une forme complémentaire de la section non circulaire.

2. Prise de distribution de fluide selon la revendication 1, **caractérisée en ce que** le passage de fluide (22) de la chambre à bille-clapet (6) est conformé pour présenter une section polygonale sur le tronçon d'entrée (28).

3. Prise de distribution de fluide selon l'une des revendications 1 ou 2, **caractérisée en ce que** le passage de fluide (22) de la chambre à bille-clapet (6) est conformé pour présenter une section hexagonale sur le tronçon d'entrée (28), en particulier une structure de type 6-pans.

4. Prise de distribution de fluide selon la revendication 1, **caractérisée en ce que** le guide-embout (8) extractible comprend un logement axial (12) à fond borgne et un élément élastique (11), ledit élément élastique (11) étant agencé entre le clapet de tête (9) et le fond borgne du logement axial (12) du guide-embout (8).

5. Prise de distribution de fluide selon la revendication 1, **caractérisée en ce que** le passage de fluide (22) de la chambre à bille-clapet (6) est aménagé coaxialement à un axe longitudinal (AA) de la chambre à bille-clapet (6).

6. Prise de distribution de fluide selon la revendication 1, **caractérisée en ce que** la chambre à bille-clapet (6) comprend de 2 à 6 expansions de parois (26) agencées à la seconde extrémité (2b), typiquement de 2 à 4 expansions de parois (26).

7. Prise de distribution de fluide selon l'une des revendications 1 ou 6, **caractérisée en ce que** l'ouverture large (25) de la chambre à bille-clapet (6) comprend une bordure circulaire, les expansions de parois (26) étant portées par ladite bordure circulaire et formées d'une pièce avec ladite bordure circulaire.

8. Prise de distribution de fluide selon la revendication 6, **caractérisée en ce que** les expansions de parois (26) de la chambre à bille-clapet (6) sont des languettes.

9. Prise de distribution de fluide selon la revendication 1, **caractérisée en ce que** le corps de prise (2) comprend un orifice proximal (4) en communication fluidique avec le passage central (3) formant le logement interne du corps de prise (2), la chambre à bille-clapet (6) et le guide-embout (8) étant conformés pour être extractibles du corps de prise (2) par ledit orifice proximal (4).

10. Prise de distribution de fluide selon la revendication 9, **caractérisée en ce que** le guide-embout (8) extractible est agencé dans le corps de prise (2) entre la chambre à bille-clapet (6) et l'orifice proximal (4).

11. Prise de distribution de fluide selon la revendication 1, **caractérisée en ce que** la chambre à bille-clapet (6) a une forme générale de coupelle comprenant un corps creux de forme générale tubulaire et une paroi avant formant la première extrémité (6a) traversée par le passage de fluide (22).

12. Prise de distribution de fluide selon la revendication 11, **caractérisée en ce que** la paroi avant et le corps creux et/ou les expansions de paroi (26) de la chambre à bille-clapet (6) sont formés d'une seule pièce.

13. Utilisation d'une prise de distribution de fluide (1) selon l'une des revendications précédentes, pour distribuer un fluide au sein d'un bâtiment hospitalier, ladite prise de distribution de fluide (1) étant montée directement ou indirectement, sur une paroi du bâtiment hospitalier et raccordée fluidiquement à une canalisation de gaz ou de vide agencée au sein dudit bâtiment hospitalier.

14. Bâtiment hospitalier comprenant :
- au moins une canalisation de fluide agencée au sein dudit bâtiment hospitalier pour y acheminer du gaz ou du vide,
- au moins une pièce comprenant une paroi,
- et au moins une prise de distribution de fluide (1) selon l'une des revendications 1 à 12, montée sur ladite paroi et raccordée fluidiquement à ladite au moins une canalisation de gaz ou de vide.

## Patentansprüche

1. Fluidverteilungsanschluss (1) zum Verteilen eines Fluids innerhalb eines Krankenhausgebäudes, welcher einen Anschlusskörper (2) von länglicher Form mit einer Längsachse AA umfasst, der axial von einem zentralen Durchgang (3) durchquert wird, der eine innere Aufnahme bildet, innerhalb von der eine herausziehbare Endstückführung (8), die ein bewegliches Kopfventil (9) aufnimmt, und eine herausziehbare Ventilkugelkammer (6) angeordnet sind, wobei die herausziehbare Endstückführung (8) außerdem eine axiale Stange (13) umfasst, die auf der hinteren Außenfläche der Endstückführung (8) vorsteht und mit der Endstückführung (8) fest verbunden ist, und wobei die herausziehbare Ventilkugelkammer (6) umfasst:
- eine Umfangswand (20), die einen inneren Raum (21) begrenzt, der eine bewegliche Ventilkugel (7) enthält, und die ein äußeres Umfangsgewinde (27) trägt, das um die Umfangswand (20) herum angeordnet ist, wobei die bewegliche Ventilkugel (7) eine Kugelform aufweist oder einen länglichen Unterteil umfasst, der von einem halbkugelförmigen Kopf überragt wird,
- ein erstes Ende (6a) mit einem Fluiddurchlass (22), der mit dem inneren Raum (21) über eine innere Öffnung (24) und mit der Außenseite der Ventilkugelkammer (6) über eine äußere Öffnung (23) in Fluidverbindung steht, und
- ein zweites Ende (6b) mit einer großen Öffnung (25), die mit dem inneren Raum (4) in Fluidverbindung steht,
und wobei:
- die Endstückführung (8) an der Ventilkugel (7) der herausziehbaren Ventilkugelkammer (6) über die axiale Stange (13), die auf der hinteren Außenfläche der Endstückführung (8) vorsteht, durch den axialen Fluiddurchlass (22) hindurch zur Anlage kommt und
- die Umfangswand (20) der herausziehbaren Ventilkugelkammer (6) derart ausgebildet ist, dass sie Wanderweiterungen (26) aufweist, die am zweiten Ende (6b) angeordnet sind, wobei die Wanderweiterungen (26) von der Umfangswand (20) weg vorstehen und radial zur Längsachse (AA) hin umgebogen sind, so dass sie die bewegliche Ventilkugel (7) dort halten, und
- der zentrale Durchgang des Anschlusskörpers (2) ein erstes Innengewinde umfasst, das mit dem in der Umfangswand (20) der Ventilkugelkammer (6) angeordneten Umfangsgewinde (27) zusammenwirkt, um eine Anbringung der Ventilkugelkammer (6) in dem Anschlusskörper (2) durch Verschraubung zu ermöglichen, **dadurch gekennzeichnet, dass**
- der axiale Fluiddurchlass (22), der im ersten Ende (6a) der herausziehbaren Ventilkugelkammer (6) angeordnet ist, derart ausgebildet ist, dass er einen nicht kreisförmigen Querschnitt auf einem Eingangsteilabschnitt (28) aufweist, der sich von der äußeren Öffnung (23) des axialen Fluiddurchlasses (22) aus und in Richtung der inneren Öffnung (24) des axialen Fluiddurchlasses (22) erstreckt, um so das Verschrauben der Ventilkugelkammer (6) im Inneren des Fluidverteilungsanschlusses mithilfe eines Werkzeugs zu ermöglichen, das eine zu dem nicht kreisförmigen Querschnitt komplementäre Form aufweist.

2. Fluidverteilungsanschluss nach Anspruch 1, **dadurch gekennzeichnet, dass** der Fluiddurchlass (22) der Ventilkugelkammer (6) derart ausgebildet ist, dass er auf dem Eingangsteilabschnitt (28) einen polygonalen Querschnitt aufweist.

3. Fluidverteilungsanschluss nach einem der Ansprüche 1 oder 2, **dadurch gekennzeichnet, dass** der Fluiddurchlass (22) der Ventilkugelkammer (6) derart ausgebildet ist, dass er auf dem Eingangsteilabschnitt (28) einen sechseckigen Querschnitt aufweist, insbesondere eine Struktur vom Typ eines Sechskants.

4. Fluidverteilungsanschluss nach Anspruch 1, **dadurch gekennzeichnet, dass** die herausziehbare Endstückführung (8) eine axiale Aufnahme (12) mit verschlossenem Boden und ein elastisches Element (11) umfasst, wobei das elastische Element (11) zwischen dem Kopfventil (9) und dem verschlossenen Boden der axialen Aufnahme (12) der Endstückführung (8) angeordnet ist.

5. Fluidverteilungsanschluss nach Anspruch 1, **dadurch gekennzeichnet, dass** der Fluiddurchlass (22) der Ventilkugelkammer (6) koaxial mit einer Längsachse (AA) der Ventilkugelkammer (6) angeordnet ist.

6. Fluidverteilungsanschluss nach Anspruch 1, **dadurch gekennzeichnet, dass** die Ventilkugelkammer (6) 2 bis 6 Wanderweiterungen (26) umfasst, die am zweiten Ende (2b) angeordnet sind, typischerweise 2 bis 4 Wanderweiterungen (26) .

7. Fluidverteilungsanschluss nach einem der Ansprüche 1 bis 6, **dadurch gekennzeichnet, dass** die große Öffnung (25) der Ventilkugelkammer (6) einen kreisförmigen Rand umfasst, wobei die Wanderweiterungen (26) von dem kreisförmigen Rand getragen werden und mit dem kreisförmigen Rand aus einem Stück ausgebildet sind.

8. Fluidverteilungsanschluss nach Anspruch 6, **dadurch gekennzeichnet, dass** die Wanderweiterungen (26) der Ventilkugelkammer (6) Zungen sind.

9. Fluidverteilungsanschluss nach Anspruch 1, **dadurch gekennzeichnet, dass** der Anschlusskörper (2) eine proximale Öffnung (4) umfasst, die mit dem zentralen Durchgang (3), der die innere Aufnahme des Anschlusskörpers (2) bildet, in Fluidverbindung steht, wobei die Ventilkugelkammer (6) und die Endstückführung (8) derart ausgebildet sind, dass sie durch die proximale Öffnung (4) aus dem Anschlusskörper (2) herausziehbar sind.

10. Fluidverteilungsanschluss nach Anspruch 9, **dadurch gekennzeichnet, dass** die herausziehbare Endstückführung (8) in dem Anschlusskörper (2) zwischen der Ventilkugelkammer (6) und der proximalen Öffnung (4) angeordnet ist.

11. Fluidverteilungsanschluss nach Anspruch 1, **dadurch gekennzeichnet, dass** die Ventilkugelkammer (6) eine allgemeine Form eines Bechers aufweist, der einen Hohlkörper von rohrförmiger allgemeiner Form und eine das erste Ende (6a) bildende vordere Wand, die von dem Fluiddurchlass (22) durchquert wird, umfasst.

12. Fluidverteilungsanschluss nach Anspruch 11, **dadurch gekennzeichnet, dass** die vordere Wand und der Hohlkörper und/oder die Wanderweiterungen (26) der Ventilkugelkammer (6) aus einem Stück ausgebildet sind.

13. Verwendung eines Fluidverteilungsanschlusses (1) nach einem der vorhergehenden Ansprüche zum Verteilen eines Fluids innerhalb eines Krankenhausgebäudes, wobei der Fluidverteilungsanschluss (1) direkt oder indirekt an einer Wand des Krankenhausgebäudes angebracht ist und mit einer Gas- oder Vakuumleitung, die innerhalb des Krankenhausgebäudes angeordnet ist, in Fluidverbindung steht.

14. Krankenhausgebäude, welches umfasst:
- mindestens eine Fluidleitung, die innerhalb des Krankenhausgebäudes angeordnet ist, um hier Gas oder Vakuum zu transportieren,
- mindestens einen Raum, der eine Wand umfasst,
- und mindestens einen Fluidverteilungsanschluss (1) nach einem der Ansprüche 1 bis 12, der an dieser Wand angebracht ist und mit der mindestens einen Gas- oder Vakuumleitung in Fluidverbindung steht.

## Claims

1. Fluid distribution outlet (1) for distributing a fluid within a hospital building, comprising an outlet body (2) of elongate shape, and of longitudinal axis AA, passed through axially by a central passage (3) forming an internal housing within which there is arranged an extractable endpiece guide (8) which houses a movable head valve (9), and an extractable ball-valve chamber (6), the extractable endpiece guide (8) further comprising an axial rod (13) protruding from the rear outer surface of the endpiece guide (8) and being integral with said endpiece guide (8), and said extractable ball-valve chamber (6) comprising:
- a peripheral wall (20) delimiting an internal compartment (21) containing a movable ball-valve (7), and carrying an outer peripheral thread (27) formed around said peripheral wall (20), the movable ball-valve (7) having a spherical shape or comprising an elongate base surmounted by a semi-spherical head,
- a first end (6a) with a fluid passage (22) in fluidic communication with the internal compartment (21) via an internal orifice (24) and with the outside of the ball-valve chamber (6) via an external orifice (23), and
- a second end (6b) with a wide opening (25) in fluidic communication with the internal compartment (4),
and wherein:
- the endpiece guide (8) comes to rest on the ball-valve (7) of the extractable ball-valve chamber (6) via the axial rod (13) protruding from the rear outer surface of the endpiece guide (8), through the axial fluid passage (22), and
- the peripheral wall (20) of the extractable ball-valve chamber (6) is shaped to have wall extensions (26) arranged at the second end (6b), said wall extensions (26) protruding a distance from said peripheral wall (20) and being folded radially towards the longitudinal axis (AA) so as to retain the movable ball-valve (7) there, and
- the central passage of the outlet body (2) comprises a first thread cooperating with the peripheral thread (27) formed in the peripheral wall (20) of the ball-valve chamber (6) in order to allow the ball-valve chamber (6) to be mounted in the outlet body (2) by screwing,
**characterized in that**
- the axial fluid passage (22) arranged in the first end (6a) of the extractable ball-valve chamber (6) is shaped to have a non-circular cross section on an inlet segment (28) extending from the outer orifice (23) of the axial fluid passage (22) and towards the inner orifice (24) of the axial fluid passage (22), in order to allow the ball-valve chamber (6) to be screwed inside the fluid distribution outlet, by means of a tool having a shape complementing the non-circular cross section.

2. Fluid distribution outlet according to Claim 1, **characterized in that** the fluid passage (22) of the ball-valve chamber (6) is shaped to have a polygonal cross section on the inlet segment (28).

3. Fluid distribution outlet according to either of Claims 1 and 2, **characterized in that** the fluid passage (22) of the ball-valve chamber (6) is shaped to have a hexagonal cross section on the inlet segment (28), in particular a structure of the hexagon socket type.

4. Fluid distribution outlet according to Claim 1, **characterized in that** the extractable endpiece guide (8) comprises an axial housing (12) with a blind bottom and an elastic element (11), said elastic element (11) being arranged between the head valve (9) and the blind bottom of the axial housing (12) of the endpiece guide (8).

5. Fluid distribution outlet according to Claim 1, **characterized in that** the fluid passage (22) of the ball-valve chamber (6) is arranged coaxially with respect to a longitudinal axis (AA) of the ball-valve chamber (6).

6. Fluid distribution outlet according to Claim 1, **characterized in that** the ball-valve chamber (6) comprises from 2 to 6 wall extensions (26) arranged at the second end (2b), typically from 2 to 4 wall extensions (26) .

7. Fluid distribution outlet according to either of Claims 1 and 6, **characterized in that** the wide opening (25) of the ball-valve chamber (6) comprises a circular edge, the wall extensions (26) being carried by said circular edge and being formed integrally with said circular edge.

8. Fluid distribution outlet according to Claim 6, **characterized in that** the wall extensions (26) of the ball-valve chamber (6) are tabs.

9. Fluid distribution outlet according to Claim 1, **characterized in that** the outlet body (2) comprises a proximal orifice (4) in fluidic communication with the central passage (3) forming the internal housing of the outlet body (2), the ball-valve chamber (6) and the endpiece guide (8) being configured to be extractable from the outlet body (2) through said proximal orifice (4) .

10. Fluid distribution outlet according to Claim 9, **characterized in that** the extractable endpiece guide (8) is arranged in the outlet body (2) between the ball-valve chamber (6) and the proximal orifice (4).

11. Fluid distribution outlet according to Claim 1, **characterized in that** the ball-valve chamber (6) has the general shape of a cup comprising a hollow body of generally tubular shape and a front wall forming the first end (6a) through which the fluid passage (22) passes.

12. Fluid distribution outlet according to Claim 11, **characterized in that** the front wall and the hollow body and/or the wall extensions (26) of the ball-valve chamber (6) are formed integrally.

13. Use of a fluid distribution outlet (1) according to one of the preceding claims, for distributing a fluid within a hospital building, said fluid distribution outlet (1) being mounted directly or indirectly on a wall of the hospital building and being fluidically connected to a gas or vacuum pipe arranged within said hospital building.

14. Hospital building comprising:
- at least one fluid pipe arranged within said hospital building in order there to convey gas or a vacuum,
- at least one room comprising a wall,
- and at least one fluid distribution outlet (1) according to one of Claims 1 to 12, mounted on said wall and fluidically connected to said at least one gas or vacuum pipe.
